# EUROPEAN PATENT SPECIFICATION

(11) **EP 3 270 161 B1**
(45) Date of publication and mention of the grant of the patent: **09.08.2023**
(21) Application number: 16764578.7
(22) Date of filing: 09.02.2016
(51) Int. Cl.: G01N 33/556, G01N 27/22, G01N 33/49, G01N 33/53

(54) **SAMPLE FOR ELECTRICAL CHARACTERISTIC MEASUREMENT, ELECTRICAL CHARACTERISTIC MEASURING METHOD, AND ELECTRICAL CHARACTERISTIC MEASURING DEVICE**
PROBE ZUR MESSUNG VON ELEKTRISCHEN EIGENSCHAFTEN, VERFAHREN ZUR MESSUNG ELEKTRISCHER EIGENSCHAFTEN UND VORRICHTUNG ZUR MESSUNG ELEKTRISCHER EIGENSCHAFTEN
ÉCHANTILLON DEVANT SERVIR À LA MESURE DE CARACTÉRISTIQUES ÉLECTRIQUES, PROCÉDÉ ET DISPOSITIF DE MESURE ASSOCIÉS

(30) Priority: 13.03.2015 JP 2015050884
(43) Date of publication of application: 17.01.2018
(73) Proprietor: Sony Group Corporation, Tokyo 108-0075 (JP)
(72) Inventor: MACHIDA, Kenzo, Tokyo 108-0075 (JP); BRUN, Marcaurele, Tokyo 108-0075 (JP); HAYASHI, Yoshihito, Tokyo 108-0075 (JP); KAWAGUCHI, Kaori, Tokyo 108-0075 (JP); LEE, Seungmin, Tokyo 108-0075 (JP)
(74) Representative: D Young & Co LLP
(86) International application number: PCT/JP2016/053762
(87) International publication number: WO 2016/147747

(56) References cited:
- EP-A1- 2 500 726
- WO-A1-85/05450
- JP-A- 2012 194 087
- US-A- 5 895 760
- US-A1- 2006 210 429
- US-B1- 6 432 657
- US-B1- 6 531 321
- P. S. VASSAR ET AL: "PHYSICOCHEMICAL EFFECTS OF ALDEHYDES ON THE HUMAN ERYTHROCYTE", THE JOURNAL OF CELL BIOLOGY : JCB, vol. 53, no. 3, June 1972 (1972-06), pages 809-818, XP055512105, US ISSN: 0021-9525, DOI: 10.1083/jcb.53.3.809
- PING YANG ET AL: "Mannitol-adenine-phosphate: a novel solution for intraoperative blood salvage : Blood Salvage Solution", TRANSFUSION., vol. 54, no. 4, April 2014 (2014-04), pages 1146-1152, XP055512413, US ISSN: 0041-1132, DOI: 10.1111/trf.12370
- OGAWA, S. ET AL.: 'Formalin-fixed Erythrocytes for Standardization of the Electronic cell counter' BULLETIN OF THE INSTITUTE OF PUBLIC HEALTH vol. 17, no. 3, September 1968, pages 257 - 262, XP009506200

## Description

### TECHNICAL FIELD

The present technology relates to a use of a sample in electrical characteristic measurement, an electrical characteristic measurement method, and an electrical characteristic measurement device.

### BACKGROUND ART

An antiplatelet aggregating agent or an anticoagulant is administered prophylactically to reduce the risk of thrombosis. This prophylactic administration can have the side effect of increasing the risk of bleeding if the dosage is excessive. In order to prevent this side effect and obtain a sufficient preventive effect, timely evaluation is made of blood coagulability of medicated subjects.

In recent years, some techniques have been developed for evaluating the degree of blood coagulation simply and accurately. For example, Patent Document 1 discloses a technique for obtaining information on blood coagulation from the permittivity of blood, and describes "a blood coagulation system analysis device including: a pair of electrodes; means for applying an alternating voltage to the pair of electrodes at predetermined time intervals; means for measuring the permittivity of blood disposed between the pair of electrodes; and means for analyzing the degree of action of the blood coagulation system using the permittivity of blood measured at the predetermined time intervals after the effect of a coagulant acting on the blood is ended".

### CITATION LIST

### PATENT DOCUMENT

Patent Document 1: Japanese Patent Application Laid-Open No. 2010-181400

A measurement of parameters of a sample comprising partially fixed erythrocytes is disclosed in P. S. VASSAR ET AL: "PHYSICOCHEMICAL EFFECTS OF ALDEHYDES ON THE HUMAN ERYTHROCYTE", THE JOURNAL OF CELL BIOLOGY : JCB, vol. 53, no. 3, 1 June 1972 (1972-06-01), pages 809-818.

### SUMMARY OF THE INVENTION

### PROBLEMS TO BE SOLVED BY THE INVENTION

At present, no attempt has been made to check the measurement accuracy of the device for measuring an electrical characteristic of blood samples as disclosed in Patent Document 1 before the measurement of blood samples from subjects. If there is something wrong with the device for measuring an electrical characteristic of blood samples or with the measurement procedure so that accurate measurement is not possible, not only the measurement itself will be useless, but also the blood samples taken from subjects will be wasted.

It is therefore a principal object of the present technology to provide a technology useful for verifying measurement accuracy before measurement of an electrical characteristic of blood samples.

### SOLUTIONS TO PROBLEMS

The invention is defined by the claims.

### EFFECTS OF THE INVENTION

The present technology makes it possible to verify measurement accuracy before measurement of an electrical characteristic of blood samples.

It will be understood that the effects described herein are non-limiting and the present technology may bring about any of the effects described herein.

### BRIEF DESCRIPTION OF DRAWINGS

Fig. 1 is a flowchart showing an example of an electrical characteristic measurement method according to a preferred embodiment of the present technology.
Fig. 2 is a block diagram showing the general configuration of an example of an electrical characteristic measurement device according to an embodiment of the present technology.
Fig. 3 is a drawing-substitute graph showing the results of measurement of temporal changes in the permittivity of samples prepared in Experiment 1 of Test Example 1.
Fig. 4 is a drawing-substitute graph showing the results of measurement of temporal changes in the permittivity of samples prepared in Experiment 2 of Test Example 1.
Fig. 5 is a drawing-substitute graph showing the results of measurement of temporal changes in the permittivity of samples prepared in Experiment 3 of Test Example 1.
Fig. 6 is a drawing-substitute graph showing the results of measurement of temporal changes in the permittivity of samples prepared in Experiment 4 of Test Example 1.
Fig. 7 is a drawing-substitute graph showing the results of measurement of temporal changes in the permittivity of samples prepared in Test Example 2.

### MODE FOR CARRYING OUT THE INVENTION

Hereinafter, preferred modes for carrying out the present technology will be described with reference to the drawings. It will be understood that the embodiments described below are typical embodiments of the present technology and should not be construed as limiting the scope of the present technology. Descriptions will be provided in the following order.
1. Sample for use in electrical characteristic measurement
   (1) Uses
   (2) Incompletely fixed erythrocytes
   (3) Fixative
   (4) Fixing process
   (5) Optional components
2. Electrical characteristic measurement method
3. Electrical characteristic measurement device

### 1. Sample for use in electrical characteristic measurement

### (1) Uses

The use of a sample according to the present technology in electrical characteristic measurement is for use in electrical characteristic measurement. The sample for use in electrical characteristic measurement is preferably for use in measuring an electrical characteristic of blood samples to be measured and is useful for verifying the measurement accuracy. Thus, hereinafter, the use of a sample according to the present technology in electrical characteristic measurement will also be referred to as the "reference sample." The reference sample is more preferably used with a device (electrical characteristic measurement device) for measuring an electrical characteristic of blood samples to be measured. The use of the reference sample makes it possible to verify the measurement accuracy of the electrical characteristic measurement device in measuring an electrical characteristic of blood samples. Therefore, the reference sample can be used as a sample for controlling the quality of the electrical characteristic measurement device, namely, what is called a quality control (QC) sample.

The electrical characteristic measurement device is preferably configured to measure an electrical characteristic of blood samples to be measured at any frequency over time. The electrical characteristic measurement device may have the function of evaluating or analyzing the condition of blood in the measured blood sample to be measured on the basis of the measured electrical characteristic.

Next, the configurations of the reference sample will be described. The electrical characteristic measurement device suitable for use with the reference sample will be described in detail later in the section titled "2. Electrical characteristic measurement device."

### (2) Incompletely fixed erythrocytes

The use of a sample according to the present technology in electrical characteristic measurement includes erythrocytes incompletely fixed with a fixative. The fixed erythrocytes can undergo temporal changes in its electrical characteristic when mixed with plasma components.

As regards the erythrocytes contained in the reference sample, the term "incompletely fixed with a fixative" means that erythrocytes are not completely fixed with a fixative. Erythrocytes completely fixed with a fixative are in such a state that they completely lose their original flexibility. In contrast, erythrocytes incompletely fixed with a fixative are in such a state that they retain a certain part of their original degree of flexibility. The degree of fixation of erythrocytes with a fixative is influenced by, for example, the fixative type, the fixative concentration, and the time and temperature of treatment of erythrocytes with the fixative. Therefore, as an example, the erythrocytes incompletely fixed with a fixative may be erythrocytes fixed with a fixative at a concentration lower than the concentration required to completely fix the erythrocytes.

Since the erythrocytes in the reference sample are incompletely fixed with a fixative, only the degradation of the erythrocytes is suppressed while their flexibility is maintained. It is known that as erythrocytes undergo degradation, they change their shape and lose their original function. However, the present technology makes it possible to suppress such degradation. On the other hand, an electrical characteristic of a mixture of plasma components and the erythrocytes incompletely fixed with a fixative can change over time. This is because the erythrocytes used in the present technology are so fixed that they can resist degradation-induced changes in shape but so incompletely fixed that they retain a certain level of flexibility necessary for serving their original function. In contrast, for example, in a case where erythrocytes are completely fixed with a fixative, an electrical characteristic of a mixture of such strongly fixed erythrocytes and plasma components hardly changes over time, because they lose a certain level of flexibility necessary for serving their original function, though they resists degradation-induced changes in shape.

The erythrocytes may be incompletely fixed to any specific extent as long as the fixed erythrocytes resist degradation-induced changes in shape while retaining a certain level of flexibility. For example, the erythrocytes may be so fixed that they can undergo rouleaux formation when mixed with plasma components. Rouleaux formation of the incompletely fixed erythrocytes makes it easy to observe temporal changes in electrical characteristic (such as an increase in permittivity) using the reference sample.

In addition, the fixed erythrocytes may also be such that when they are mixed with plasma components, a predetermined characteristic value of an electrical characteristic of the mixture at a predetermined frequency will remain within a predetermined range for a certain period. More specifically, for example, the fixed erythrocytes may be such that when they are mixed with plasma components, a predetermined characteristic value of an electrical characteristic of the mixture at a predetermined frequency will remain within the range of ± 20% for 25 days, preferably 30 days, after the fixation.

The term "a predetermined characteristic value of an electrical characteristic" may refer to, for example, only a predetermined value such as a maximum or minimum value of permittivity, and may also refer to a predetermined rate of change, such as the difference between maximum and minimum values of permittivity. More specifically, for example, the erythrocytes may be so fixed that the rate of change from A to B falls within the range of ± 20% in which A is the maximum value of the permittivity on day 0 after the fixation, and B is the maximum value of the permittivity on day 30 after the fixation. In addition, for example, the erythrocytes may be so fixed that the rate of change from C to D falls within the range of ± 20% in which C is the difference between the maximum and minimum values of the permittivity on day 0 after the fixation, and D is the difference between the maximum and minimum values of the permittivity on day 30 after the fixation.

Now, preferred modes of the fixed erythrocytes will be described with reference to an example where the reference sample is subjected to measurement of permittivity, as an electrical characteristic at any frequency, over time.

The erythrocytes are preferably so fixed with a fixative that when they are mixed with plasma components, the difference between the maximum and minimum values of the permittivity of the mixture measured at a frequency of 10 MHz or 1 MHz over time will remain within the range of ± 20%, preferably within the range of ± 10%, for 25 days, preferably for 30 days, after the fixation. The fixation in this manner makes it easy to observe changes in electrical characteristic while maintaining a certain level of storage stability and thus makes it possible to more advantageously use the reference sample according to the present technology as a QC sample.

In addition, for example, the erythrocytes may also be so fixed that when they are mixed with plasma components, the rate of temporal change in an electrical characteristic of the mixture at a predetermined frequency will fall within a predetermined range. More specifically, the erythrocytes may be so fixed that when they are mixed with plasma components, the rate of change between the minimum and maximum values of an electrical characteristic of the mixture measured at a predetermined frequency over time will be at least a predetermined value.

Now, preferred modes of the fixed erythrocytes will be described with reference to an example where the reference sample is subjected to measurement of permittivity, as an electrical characteristic at any frequency, over time.

The erythrocytes are preferably so fixed that when they are mixed with plasma components, the rate of change between the minimum and maximum values of the permittivity of the mixture measured at a frequency of 10 MHz over time will be 6% or more. The fixation in this manner makes it easy to observe changes in electrical characteristic and thus makes it possible to more advantageously use the reference sample according to the present technology as a QC sample.

### (3) Fixative

The fixative may be of any type capable of incompletely fixing erythrocytes. The fixative may be, for example, any of an aldehyde, an oxazolidine, an alcohol, and a cyclic urea. Preferred examples of the fixative include formaldehyde, glutaraldehyde, 2-propenal, diazolidinyl urea, imidazolidinyl urea, dimethylol urea, and 2-bromo-2-nitropropane-1,3-diol. One, two, or more of these fixatives may be used alone or in the form of a mixture. Among these fixatives, glutaraldehyde is more preferred because erythrocytes can be easily fixed with it.

When used, these fixatives are preferably diluted with, for example, a buffer solution such as phosphate buffered saline (hereinafter sometimes abbreviated as "PBS") or a physiological saline solution so that erythrocytes can be easily incompletely fixed with the fixative or erythrocytes can fail to be completely fixed with the fixative. In view of ease of handling, therefore, as the fixative, a fixative-containing composition is preferably used, which is in the form of a solution containing the fixative as a fixative component. When the fixative is diluted and used, the concentration of the fixative can be easily adjusted, and the degree of fixation of erythrocytes can be easily adjusted by changing the concentration of the fixative. In this regard, the diluent may be of any type that can make the salt concentration and pH close to those of blood and is not reactive with the fixative.

The concentration of the fixative used for fixing erythrocytes is preferably, but not particularly limited to, 0.001% by weight or more, more preferably 0.006% by weight or more, still more preferably 0.01% by weight or more, in order to improve the storage stability while fixing erythrocytes. On the other hand, erythrocytes should not be completely fixed and should be incompletely fixed so that when they are mixed with plasma components, an electrical characteristic of the mixture can change over time. From these points of view, the concentration of the fixative is preferably 0.05% by weight or less, more preferably 0.025% by weight or less. For example, glutaraldehyde suitable as the fixative may be used at a concentration in the above range. However, regardless of the above range, the concentration of the fixative may be appropriately adjusted depending on the fixative type or to what extent erythrocytes should be fixed. In addition, the degree of fixation can also be adjusted by adjusting the reaction time and temperature when the erythrocytes are fixed with the fixative.

### (4) Fixing process

As a non-limiting example, the method of fixing erythrocytes with the fixative preferably includes the steps of removing plasma and leukocytes (buffy coats) from blood, washing the resulting erythrocytes, and adding the fixative to the washed erythrocytes.

A specific example of the step of removing plasma and leukocytes (buffy coats) from blood may include centrifuging blood and then removing the resulting supernatant containing plasma and leukocytes (buffy coats). Specifically, the step of washing the resulting erythrocytes may include adding a buffer solution such as phosphate buffered saline (PBS) to the erythrocytes, mixing them by inversion, and removing the supernatant. This washing step is preferably repeated several times.

Specifically, the step of adding the fixative to the washed erythrocytes includes adding PBS to the washed erythrocytes so that a certain hematocrit value of about several % can be obtained, and then adding, to the mixture, a dilution obtained by diluting the fixative to a certain concentration with PBS so that the fixative is added at such a concentration that the erythrocytes are incompletely fixed. More specifically, for example, a dilution obtained by diluting glutaraldehyde to about 2.5% with PBS may be used as the fixative.

After the fixative is added to the erythrocytes, the mixture is allowed to stand at room temperature (e.g., 15 to 30°C), preferably for about 1 to 30 minutes, more preferably for about 1 to 10 minutes, so that fixation reaction is allowed to proceed. Subsequently, the mixture is preferably subjected to the steps of centrifuging the mixture, removing the resulting supernatant, and washing the fixed erythrocytes. At this stage, the washing step may include, similarly to the above, adding a buffer solution such as PBS to the fixed erythrocytes, mixing them by inversion, further centrifuging the mixture, and removing the supernatant. This washing step is also preferably repeated several times. A mannitol adenine phosphate (MAP) solution may be added to the fixed erythrocytes that have undergone the final washing step, and the resulting mixture may be stored (e.g., stored under refrigeration). In this case, the amount of the MAP solution may be, for example, about half of the volume of the washed fixed erythrocytes.

### (5) Optional components

The reference sample may contain, in addition to the erythrocytes fixed with the fixative, a diluent useful for diluting the fixative as mentioned above, such as PBS or any other buffer solution or a physiological saline solution. In addition, the reference sample may also contain a preservative solution suitable for use in storing the erythrocytes fixed in the fixation process described above. One, two, or more known preservative solutions may be freely selected and used as the preservative solution as long as the effect of the present technology is not impaired. Examples of the preservative solution include an ACD solution containing predetermined amounts of sodium citrate hydrate, citric acid hydrate, and glucose, a CPD solution containing predetermined amounts of sodium citrate hydrate, citric acid hydrate, glucose, and sodium dihydrogen phosphate, a MAP solution, and an AIS solution containing adenine, inosine, and sucrose. These preservative solutions are all commercially available. Among them, the MAP solution is preferred because it is suitable for storage of erythrocytes. In this regard, the MAP solution is a preservative solution containing predetermined amounts of D-mannitol, adenine, sodium dihydrogen phosphate, sodium citrate hydrate, citric acid hydrate, glucose, and sodium chloride. In a case where the MAP solution is used as the preservative solution, the fixed erythrocytes are dispersed in the MAP solution to form a sample for use in electrical characteristic measurement.

In this regard, the sample for the use according to the present technology in electrical characteristic measurement may also be provided in the form of a kit separately including: the erythrocytes fixed with the fixative; a diluent, such as a buffer solution, for diluting the fixative; the optional components described above; and plasma components (a sample kit for use in electrical characteristic measurement). More preferably, the sample kit for use in electrical characteristic measurement includes, separately, a mixture of the fixed erythrocytes and the preservative solution, the diluent, the plasma components, and, if necessary, the optional components described above. The sample kit for use in electrical characteristic measurement, which includes the respective components separately, is advantageous in that when the kit is used in electrical characteristic measurement, an adjustment can be easily made in such a manner that temporal changes in electrical characteristic can be achieved depending on, for example, the device used in the measurement.

### 2. Electrical characteristic measurement method

The electrical characteristic measurement method according to the present technology is a method of measuring an electrical characteristic of the reference sample over time before the electrical characteristic of a blood sample is measured over time. In this method, the accuracy of the electrical characteristic measurement can be verified using the reference sample before the blood sample is subjected to measurement. Therefore, the measurement of the blood sample after the verification makes it possible to obtain highly reliable measurement data. The measurement of the reference sample and the blood sample is preferably performed using an electrical characteristic measurement device such as a dielectric coagulometer.

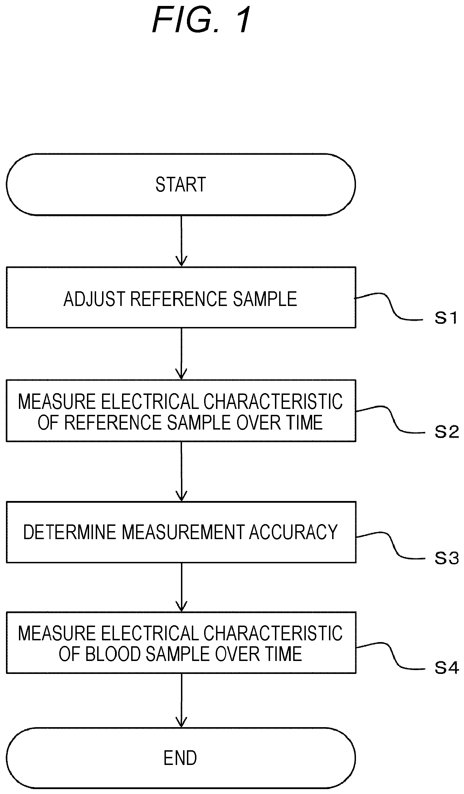

Fig. 1 is a flowchart showing an example of the electrical characteristic measurement method according to a preferred embodiment of the present technology. As described above, the electrical characteristic measurement method according to the present technology includes a first measurement step S2 including measuring an electrical characteristic of the reference sample over time. As shown in Fig. 1, this measurement method preferably includes an adjustment step S1 including adjusting the reference sample before the first measurement step and a determination step S3 including determining, after the first measurement step S2, the accuracy of the measurement on the basis of data on temporal changes in the electrical characteristic of the reference sample obtained in the first measurement step S2. In addition, after the measurement accuracy is verified to be acceptable in the determination step S3, a second measurement step S4 is preferably performed including measuring the electrical characteristic of a blood sample over time.

The electrical characteristic measured in the first and second measurement steps S2 and S4 and the electrical characteristic used in the determination step S3 may be an electrical characteristic at any frequency. Examples of the electrical characteristic include impedance, conductance, admittance, capacitance, permittivity, conductivity, phase angle, and quantities obtained by conversion of these electrical quantities. One, two, or more of these electrical characteristics may be measured and used.

In the adjustment step S1, the reference sample may be so adjusted that it can clearly change in electrical characteristic over time when its electrical characteristic is measured over time. Specifically, the reference sample may be adjusted, for example, by mixing the reference sample (fixed erythrocytes) with plasma components or by adding, to the mixture liquid of the reference sample and plasma components, an additive for strengthening temporal changes in electrical characteristic, such as an anticoagulant or a blood coagulation factor. The plasma components may be frozen plasma or freeze-dried plasma. In addition, examples of the additive that may be used include heparins such as heparin and low molecular weight heparins, coumarin compounds such as warfarin, fibrinogen, fibrin, prothrombin, thrombin, thromboplastin, and inhibitors thereof.

Performing the adjustment step S1 makes it possible to control the degree of temporal change in the electrical characteristic of the reference sample and to obtain data on temporal change in the electrical characteristic closer to that in the electrical characteristic of the blood sample. From this point of view, heparin and/or fibrinogen is more preferably added to the mixture liquid containing the fixed erythrocytes, the MAP solution, and the plasma components. For example, the amount of heparin added to the mixture liquid is preferably, but not particularly limited to, 0.05 to 5% by weight, more preferably 0.1 to 1% by weight. In addition, the amount of fibrinogen added to the mixture liquid is preferably, but not particularly limited to, 0.1 to 20 mg/mL, more preferably 0.5 to 5 mg/mL.

Specifically, the determination step S3 may include determining whether or not there is a problem with the measurement accuracy by observing whether or not sufficient changes in electrical characteristic occur over time on the basis of data on temporal changes in the electrical characteristic of the reference sample obtained in the first measurement step S2. In addition, the determination step S3 may also include determining whether or not there is a problem with the measurement accuracy on the basis of the difference obtained by comparing a predetermined reference value and data on temporal changes in the electrical characteristic of the reference sample obtained in the first measurement step S2.

In this regard, the electrical characteristic measurement method according to the present technology may be stored in the form of a program in a personal computer or a hardware resource including a control unit including, for example, a CPU, a recording medium (such as a nonvolatile memory (such as a USB memory), HDD, or CD), and other components, and may be executed by the personal computer or the control unit.

### 3. Electrical characteristic measurement device

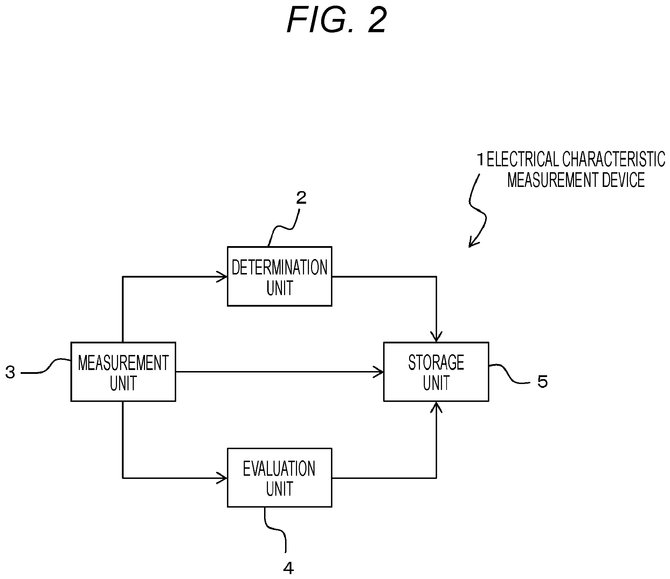

The electrical characteristic measurement device according to the present technology includes a determination unit that determines measurement accuracy on the basis of a predetermined reference value and measured values based on the electrical characteristic measured over time using the reference sample. Fig. 2 is a block diagram showing the general configuration of an electrical characteristic measurement device 1 as an example of the electrical characteristic measurement device according to an embodiment of the present technology. The electrical characteristic measurement device 1 preferably includes, in addition to the determination unit 2, a measurement unit 3, an evaluation unit 4, a storage unit 5, a display unit (not shown), and other components.

### (1) Determination unit

The determination unit 2 determines measurement accuracy on the basis of a predetermined reference value and electrical characteristic values measured over time for the reference sample. For example, the measurement accuracy may be determined by comparing a predetermined reference value and electrical characteristic values of the reference sample measured over time and determining whether the measured values are normal or abnormal on the basis of the differences between the measured values and the reference value. The user of the device can check whether there is something wrong with the device or the reference sample in a case where the determination unit 2 indicates low measurement accuracy, such as an abnormal measured value, as a result of determination of the measurement accuracy.

Before a blood sample is subjected to measurement, the measurement accuracy of the electrical characteristic measurement device 1 can be checked by measuring an electrical characteristic of the reference sample over time using the device 1 having the determination unit 2. Therefore, highly reliable measurement data can be obtained for the blood sample. In this regard, the reference value may vary within a certain numerical value range. In addition, the reference value may be stored in the storage unit 5 of the electrical characteristic measurement device 1 or in an external storage device or other devices.

The electrical characteristic used in the determination unit 2 is data measured by the measurement unit 3 provided inside the electrical characteristic measurement device 1 or by an external measurement device. Examples of the electrical characteristic include impedance, conductance, admittance, capacitance, permittivity, conductivity, phase angle, and quantities obtained by conversion of these electrical quantities. One, two, or more electrical characteristics may be used in the determination unit 2.

### (2) Measurement unit

The measurement unit 3 measures an electrical characteristic of the reference sample and the blood sample over time at a specific frequency or in a specific frequency band. The electrical characteristic measurement device 1 does not need to include the measurement unit 3. In such a case, data measured using an external electrical characteristic measurement device may be used. Specific examples of the electrical characteristic measured by the measurement unit 3 are similar to those described above for the determination unit 2. The measurement unit 3 and so on may measure one, two, or more electrical characteristics. In this regard, the electrical characteristic measurement device 1 may be configured to remove noise from the data on temporal changes in the electrical characteristic measured by, for example, the measurement unit 3 using a data processing unit provided inside the device 1 or using an external data processing device.

The measurement unit 3 may have one or more sample holding sections (not shown) into which the reference sample or the blood sample is to be injected at the time of measurement. The electrical characteristic measurement device 1 does not need to have the sample holding section, and the measurement unit 3 may also be so designed that, for example, a known cartridge type measurement container can be attached to it. The measurement unit 3 may be configured to measure the impedance or permittivity of the reference sample and the blood sample by applying an AC voltage between a pair of electrodes provided in the sample holding section. In this case, an impedance analyzer or a network analyzer may also be used as the measurement unit.

### (3) Evaluation unit

The evaluation unit 4 evaluates the condition of blood on the basis of data on temporal changes in the electrical characteristic of the blood sample. Examples of the condition of blood to be evaluated include a blood coagulation state, a blood component coagulation state, an erythrocyte sedimentation state, an erythrocyte rouleaux formation state, and a clot retraction state. The electrical characteristic for use in the evaluation by the evaluation unit 4 may be the above electrical characteristic at any frequency (e.g., a frequency of 1 kHz to 50 MHz) depending on the blood condition to be predicted or detected.

### (4) Storage unit

The electrical characteristic measurement device 1 may include a storage unit 5 stores the results of measurement of the reference sample and the blood sample by the measurement unit 3 or the like, the reference value for comparison with the measured value of the reference sample, the result of evaluation by the evaluation unit 4, and other information. In the electrical characteristic measurement device according to the present technology, the storage unit 5 is not indispensable and may be replaced by an external storage device connected thereto. The storage unit 5 may be, for example, a hard disk.

In addition, the display unit displays the data on temporal changes in the electrical characteristic of the reference sample and the blood sample measured by the measurement unit or the like, the result of evaluation by the evaluation unit, and other information.

In addition, the electrical characteristic measurement device according to the present technology may be used as a part of a system connected to, for example, a server and a user interface via a network.

### [Examples]

Hereinafter, the present technology will be described in more detail with reference to test examples. It will be understood that the test examples described below should not be construed as limiting the scope of the present technology.

### <Test Example 1>

### [Experiment 1]

Blood taken in advance from healthy persons was centrifuged, and the resulting supernatant plasma was removed. The resulting erythrocytes were washed by adding phosphate buffered saline (PBS) to the blood product (erythrocytes) obtained by removing plasma and buffy coats from the blood, mixing them by inversion, then centrifuging the resulting mixture, and removing the resulting supernatant. This erythrocyte washing step was repeated twice. PBS was added to the washed erythrocytes to achieve a hematocrit value of about 5%. Subsequently, a 2.5% by weight dilution of glutaraldehyde in PBS was added to the mixture to reach each of the concentrations below.
(1a) Glutaraldehyde: 0% by weight (control without fixation)
(1b) Glutaraldehyde: 0.013% by weight
(1c) Glutaraldehyde: 0.025% by weight

After glutaraldehyde was added, the mixture was allowed to stand at room temperature for 5 minutes. Subsequently, the mixture was centrifuged, and the resulting supernatant was removed. Subsequently, a washing step was performed, including adding PBS to the erythrocytes fixed with glutaraldehyde at each of the concentrations (1b) and (1c) (hereinafter referred to as the "fixed erythrocytes") and to the erythrocytes not fixed with glutaraldehyde (1a) (hereinafter referred to as the "unfixed erythrocytes"), respectively, mixing them by inversion, centrifuging the mixture, and removing the resulting supernatant. This washing step was repeated several times. A MAP solution in a volume equal to half of that of the washed fixed or unfixed erythrocytes was added to the washed fixed erythrocytes and the washed unfixed erythrocytes, respectively. The resulting mixtures were stored under refrigeration to give reference samples 1a, 1b, and 1c.

### [Experiment 2]

In order to confirm the reproducibility, reference samples 2a, 2b, and 2c were prepared by the same procedure as in Experiment 1, except that the blood used was different from that used in Experiment 1. Reference samples 2a to 2c prepared in Experiment 2 have the following glutaraldehyde concentrations, respectively.
(2a) Glutaraldehyde: 0% by weight (control without fixation)
(2b) Glutaraldehyde: 0.013 % by weight
(2c) Glutaraldehyde: 0.025 % by weight

### [Experiment 3]

In order to confirm the reproducibility, reference samples 3a, 3b, and 3c were prepared by the same procedure as in Experiment 1, except that the blood used was different from that used in Experiments 1 and 2. Reference samples 3a to 3c prepared in Experiment 3 have the following glutaraldehyde concentrations, respectively.
(3a) Glutaraldehyde: 0% by weight (control without fixation)
(3b) Glutaraldehyde: 0.006% by weight
(3c) Glutaraldehyde: 0.013 % by weight

### [Experiment 4]

In order to confirm the reproducibility, reference samples 4a, 4b, and 4c were prepared by the same procedure as in Experiment 1, except that the blood used was different from that used in Experiments 1 to 3. Reference samples 4a to 4c prepared in Experiment 4 have the following glutaraldehyde concentrations, respectively.
(4a) Glutaraldehyde: 0% by weight (control without fixation)
(4b) Glutaraldehyde: 0.006% by weight
(4c) Glutaraldehyde: 0.013 % by weight

Each of the reference samples prepared in Experiments 1 to 4 was adjusted as described below before subjected to electrical characteristic measurement.

Freeze-dried plasma was redissolved in a specified amount of water. The erythrocytes stored with the MAP solution in each reference sample (each of the fixed and unfixed erythrocyte samples) and the redissolved plasma were mixed in a ratio of 1 : 1 (volume ratio) to form a mixture liquid. An aqueous calcium solution as a reagent was dispensed into the sample holder of a dielectric coagulometer, and each mixture liquid was injected into the sample holder and then subjected to measurement of temporal changes in permittivity in the frequency range of about 100 Hz to 40 MHz.

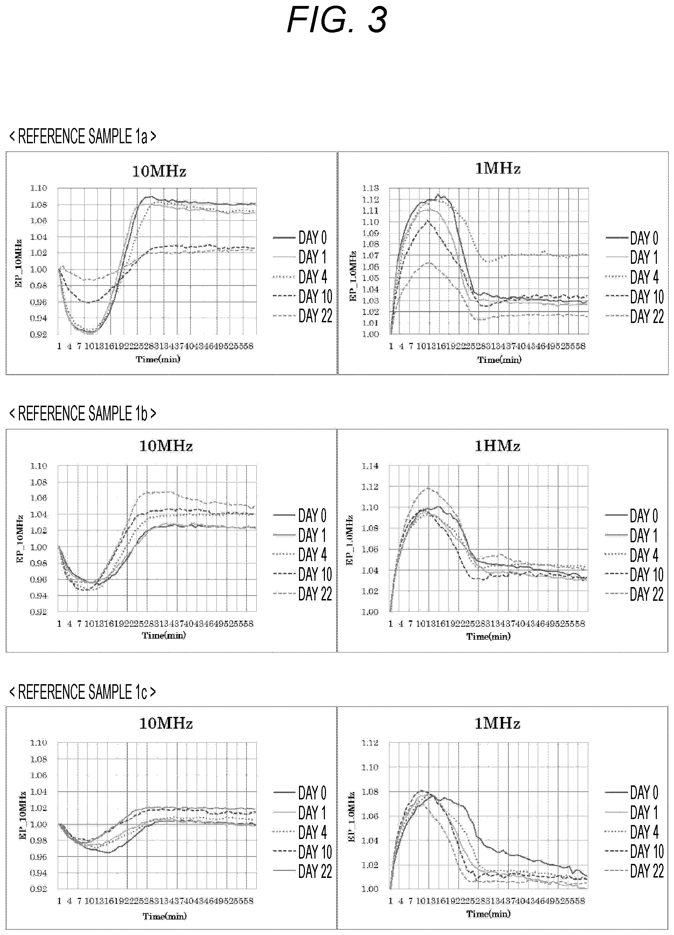

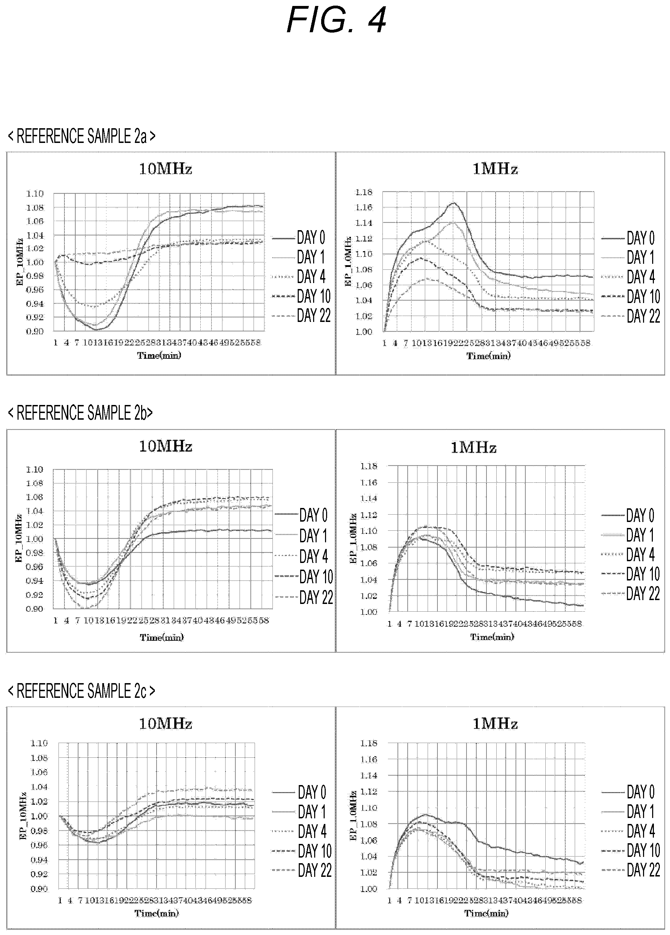

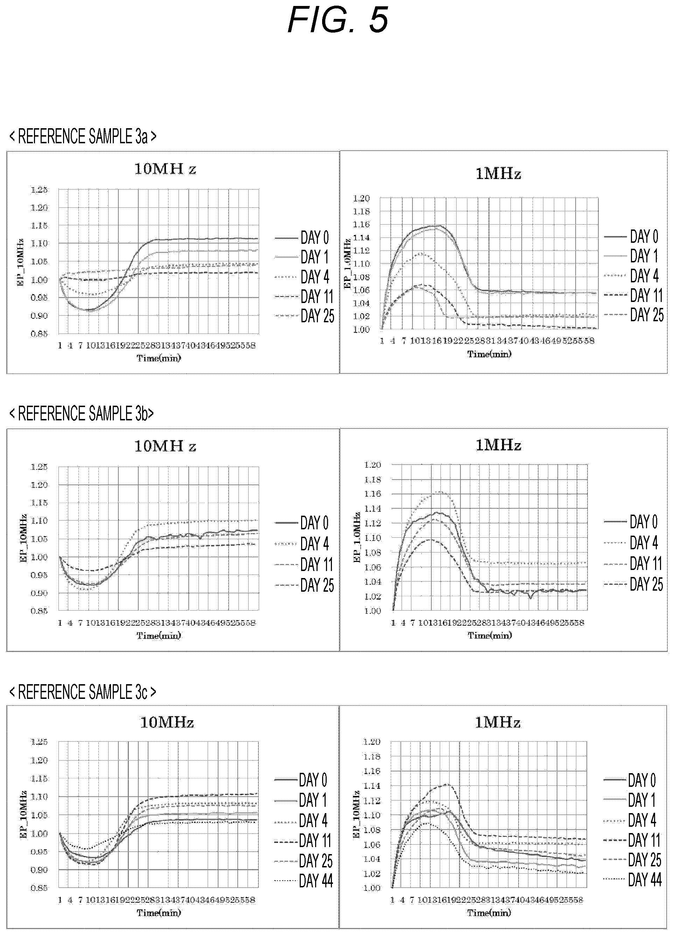

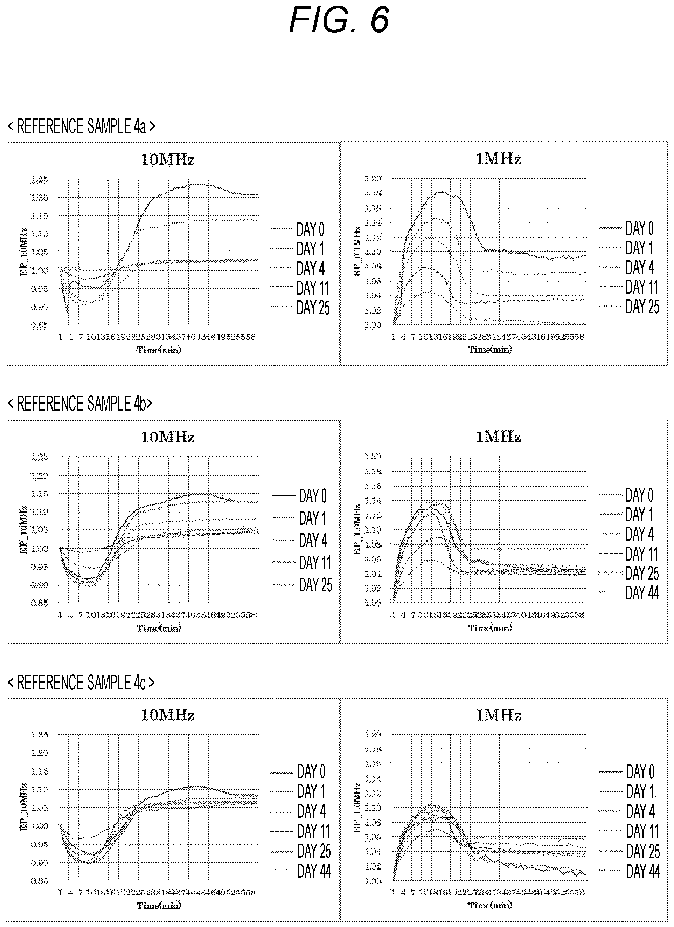

Fig. 3 shows the results of measurement of reference samples 1a to 1c prepared in Experiment 1. Fig. 4 shows the results of measurement of reference samples 2a to 2c prepared in Experiment 2. Fig. 5 shows the results of measurement of reference samples 3a to 3c prepared in Experiment 3. Fig. 6 shows the results of measurement of reference samples 4a to 4c prepared in Experiment 4. Note that Figs. 3 to 6 all show the results of measurement at frequencies of 1 MHz and 10 MHz and that the horizontal axis of the graph represents time (s) while the vertical axis represents the value obtained by normalizing the permittivity with its initial value.

The results of measurement of temporal changes in permittivity for each reference sample showed that there was a clear difference between the maximum and minimum values at a frequency of 10 MHz on the basis of the permittivity values measured on day 0 and day 22 or day 44 of storage with respect to reference samples 1b and 1c, reference samples 2b and 2c, reference samples 3b and 3c, and reference samples 4b and 4c, so that temporal changes in the value based on the measured permittivity were easily observable even after the storage.

In addition, focusing on the content of glutaraldehyde, reference samples 1b, 2b, 3c, and 4c with a glutaraldehyde concentration of 0.013% by weight showed that the rate of change between the difference between the maximum and minimum values of the permittivity measured at a frequency of 10 MHz on day 0 of storage and the difference between the maximum and minimum values of the permittivity measured at a frequency of 10 MHz on any of days 1 to 25 of storage remained within the range of ± 20%.

Reference samples 3b and 4b with a glutaraldehyde concentration of 0.006% by weight showed that the rate of change between the difference between the maximum and minimum values of the permittivity measured at a frequency of 10 MHz on day 0 of storage and the difference between the maximum and minimum values of the permittivity measured at a frequency of 10 MHz on day 25 of storage exceeded the range of ± 20% whereas the rate of change between the difference between the maximum and minimum values of the permittivity measured at a frequency of 10 MHz on day 0 of storage and the difference between the maximum and minimum values of the permittivity measured at a frequency of 10 MHz on any of days 1 to 11 of storage remained within the range of ± 20%.

Reference samples 1c and 2c with a glutaraldehyde concentration of 0.025% by weight showed that the rate of change between the difference between the maximum and minimum values of the permittivity measured at a frequency of 10 MHz on day 0 of storage and the difference between the maximum and minimum values of the permittivity measured at a frequency of 10 MHz on any of days 1 to 25 of storage remained within the range of ± 20%. In the first place, however, the difference itself between the maximum and minimum values of the permittivity was small for reference samples 1c and 2c.

The above results suggest that in cases where erythrocytes are fixed with glutaraldehyde at a concentration of less than 0.006%, the fixation may be too weak so that the fixed erythrocytes may undergo degradation during storage.

On the other hand, it is suggested that in cases where erythrocytes are fixed with glutaraldehyde at a concentration of more than 0.025% by weight, the fixation may be too strong to allow them to serve their original function, so that the electrical characteristic of a mixture of them and plasma components may be less changeable over time.

Thus, it has been found that the concentration of glutaraldehyde used as a fixative should preferably be 0.006% by weight or more and 0.025% by weight or less.

On the other hand, reference samples 1a, 2a, 3a, and 4a containing erythrocytes not fixed with any fixative such as glutaraldehyde all showed large temporal changes (signals) at the initial stage (day 0 of storage) but showed a signal reduction after a lapse of several to 10 days. Thus, it has been found that reference samples 1a, 2a, 3a, and 4a are difficult to use for verification of measurement accuracy before the measurement of an electrical characteristic of blood samples.

### <Test Example 2>

Freeze-dried plasma was redissolved in a specified amount of water. The fixed erythrocytes stored with the MAP solution in reference sample 1b, which was prepared in Experiment 1 of Test Example 1, and the redissolved plasma were mixed in a ratio of 1 : 1 (volume ratio) to form a mixture liquid, in a manner similar to the above-described manner. The mixture liquid itself was named reference sample 5a. Reference samples 5b, 5c, and 5d were prepared by adding, to the mixture liquid, heparin and/or fibrinogen in the amounts shown below, respectively.
(5a) No addition
(5b) Heparin was added in an amount of 0.5% by volume to the mixture liquid.
(5c) Heparin and fibrinogen were added in amounts of 0.5% by volume and 2 mg/mL, respectively, to the mixture liquid.
(5d) Fibrinogen was added in an amount of 2 mg/mL to the mixture liquid.

Using a method similar to that in Test Example 1 with a dielectric coagulometer, reference samples 5a to 5d were subjected to measurement of temporal changes in permittivity in the frequency range of about 100 Hz to 40 MHz.

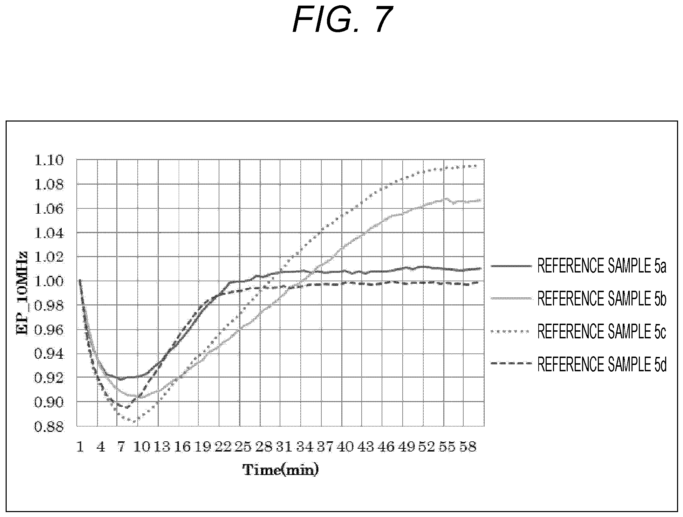

Fig. 7 shows the results. Fig. 7 shows the results of measurement at a frequency of 10 MHz, in which the horizontal axis of the graph represents time (s) while the vertical axis represents the value obtained by normalizing the permittivity with its initial value.

Reference sample 5b showed an increased coagulation time and an increased signal intensity as compared with reference sample 5a. In addition, reference sample 5c showed a further increased signal intensity. These results have shown that the addition of heparin or fibrinogen makes it possible to adjust temporal changes (signals) in electrical characteristic (permittivity).

### REFERENCE SIGNS LIST

- S1: Adjustment step
- S2: First measurement step
- S3: Determination step
- S4: Second measurement step
- 1: Electrical characteristic measurement device
- 2: Determination unit
- 3: Measurement unit
- 4: Evaluation unit
- 5: Storage unit

## Claims

1. Use of a sample in electrical characteristic measurement over time, the sample comprising
erythrocytes incompletely fixed with a fixative,
wherein the fixed erythrocytes is such that an electrical characteristic of a mixture of the fixed erythrocytes and plasma components can change over time.

2. The use of a sample in electrical characteristic measurement according to claim 1, wherein the fixed erythrocytes are such that a mixture of the fixed erythrocytes and plasma components can undergo rouleaux formation.

3. The use of a sample in electrical characteristic measurement according to claim 1, wherein the erythrocytes are so fixed that a predetermined characteristic value of an electrical characteristic of a mixture of the fixed erythrocytes and plasma components at a predetermined frequency will remain within a predetermined range for a certain period.

4. The use of a sample in electrical characteristic measurement according to claim 3, wherein the erythrocytes are so fixed that a predetermined characteristic value of an electrical characteristic of a mixture of the fixed erythrocytes and plasma components at a predetermined frequency will remain within a range of ± 20% for 25 days after fixation.

5. The use of a sample in electrical characteristic measurement according to claim 4, wherein the erythrocytes are so fixed that a difference between maximum and minimum values of permittivity of a mixture of the fixed erythrocytes and plasma components measured at a frequency of 10 MHz over time will remain within a range of ± 20% for 25 days after fixation.

6. The use of a sample in electrical characteristic measurement according to claim 1, wherein the fixative comprises glutaraldehyde.

7. The use of a sample in electrical characteristic measurement according to claim 1, the sample further comprising a MAP solution.

8. An electrical characteristic measurement method comprising:
measuring an electrical characteristic of a sample for use in electrical characteristic measurement over time before measuring an electrical characteristic of a blood sample over time, wherein
the sample for use in electrical characteristic measurement comprises erythrocytes incompletely fixed with a fixative, and the fixed erythrocytes are such that an electrical characteristic of a mixture of the fixed erythrocytes and plasma components can change over time.

9. An electrical characteristic measurement device comprising: a determination unit configured to determine measurement accuracy on the basis of a predetermined reference value and measured values based on an electrical characteristic measured over time using a sample for use in electrical characteristic measurement, wherein
the sample for use in electrical characteristic measurement comprises erythrocytes incompletely fixed with a fixative, and the fixed erythrocytes are such that an electrical characteristic of a mixture of the fixed erythrocytes and plasma components can change over time.

## Patentansprüche

1. Verwendung einer Probe bei der Messung von elektrischen Eigenschaften im Zeitverlauf, wobei die Probe umfasst
Erythrozyten, die mit einem Fixiermittel unvollständig fixiert sind,
wobei die fixierten Erythrozyten derart sind, dass sich eine elektrische Eigenschaft eines Gemisches aus den fixierten Erythrozyten und Plasmakomponenten im Zeitverlauf verändern kann.

2. Verwendung einer Probe bei der Messung von elektrischen Eigenschaften nach Anspruch 1, wobei die fixierten Erythrozyten derart sind, dass ein Gemisch aus den fixierten Erythrozyten und Plasmakomponenten Rouleaux-Bildung unterliegen kann.

3. Verwendung einer Probe bei der Messung von elektrischen Eigenschaften nach Anspruch 1, wobei die Erythrozyten derart fixiert sind, dass ein vorbestimmter Eigenschaftswert einer elektrischen Eigenschaft eines Gemisches aus den fixierten Erythrozyten und Plasmakomponenten mit einer vorbestimmten Frequenz für einen bestimmten Zeitraum in einem vorbestimmten Bereich bleiben wird.

4. Verwendung einer Probe bei der Messung von elektrischen Eigenschaften nach Anspruch 3, wobei die Erythrozyten derart fixiert sind, dass ein vorbestimmter Eigenschaftswert einer elektrischen Eigenschaft eines Gemisches aus den fixierten Erythrozyten und Plasmakomponenten mit einer vorbestimmten Frequenz für 25 Tage nach Fixierung in einem Bereich von ± 20% bleiben wird.

5. Verwendung einer Probe bei der Messung von elektrischen Eigenschaften nach Anspruch 4, wobei die Erythrozyten derart fixiert sind, dass ein Unterschied zwischen einem Höchst- und einem Mindestwert der Permittivität eines Gemisches aus den fixierten Erythrozyten und Plasmakomponenten, die mit einer Frequenz von 10 MHz im Zeitverlauf gemessen wurden, für 25 Tage nach Fixierung in einem Bereich von ± 20% bleiben wird.

6. Verwendung einer Probe bei der Messung von elektrischen Eigenschaften nach Anspruch 1, wobei das Fixiermittel Glutaraldehyd umfasst.

7. Verwendung einer Probe bei der Messung von elektrischen Eigenschaften nach Anspruch 1, wobei die Probe ferner eine MAP-Lösung umfasst.

8. Verfahren zur Messung elektrischer Eigenschaften, umfassend:
Messen einer elektrischen Eigenschaft einer Probe zur Verwendung bei der Messung von elektrischen Eigenschaften im Zeitverlauf vor dem Messen einer elektrischen Eigenschaft einer Blutprobe im Zeitverlauf, wobei
die Probe zur Verwendung bei der Messung von elektrischen Eigenschaften Erythrozyten umfasst, die mit einem Fixiermittel unvollständig fixiert sind, und die fixierten Erythrozyten derart fixiert sind, dass sich eine elektrische Eigenschaft eines Gemisches aus den fixierten Erythrozyten und Plasmakomponenten im Zeitverlauf verändern kann.

9. Vorrichtung zur Messung elektrischer Eigenschaften, umfassend:
eine Bestimmungseinheit, die dazu ausgelegt ist, die Messgenauigkeit auf Basis eines vorbestimmten Referenzwertes und gemessener Werte basierend auf einer elektrischen Eigenschaft zu bestimmen, die im Zeitverlauf mithilfe einer Probe zur Verwendung bei der Messung von elektrischen Eigenschaften gemessen wurde, wobei
die Probe zur Verwendung bei der Messung von elektrischen Eigenschaften Erythrozyten umfasst, die mit einem Fixiermittel unvollständig fixiert sind, und die fixierten Erythrozyten derart fixiert sind, dass sich eine elektrische Eigenschaft eines Gemisches aus den fixierten Erythrozyten und Plasmakomponenten im Zeitverlauf verändern kann.

## Revendications

1. Utilisation d'un échantillon dans une mesure de caractéristique électrique dans le temps, l'échantillon comprenant
des érythrocytes incomplètement fixés avec un fixateur,
dans laquelle les érythrocytes fixés sont tels qu'une caractéristique électrique d'un mélange des érythrocytes fixés et de composants plasmatiques peut varier dans le temps.

2. Utilisation d'un échantillon dans une mesure de caractéristique électrique selon la revendication 1, dans laquelle les érythrocytes fixés sont tels qu'un mélange des érythrocytes fixés et de composants plasmatiques peut subir une formation de rouleaux.

3. Utilisation d'un échantillon dans une mesure de caractéristique électrique selon la revendication 1, dans laquelle les érythrocytes sont fixés de telle sorte qu'une valeur de caractéristique prédéterminée d'une caractéristique électrique d'un mélange des érythrocytes fixés et de composants plasmatiques à une fréquence prédéterminée restera à l'intérieur d'une fourchette prédéterminée pendant une certaine durée.

4. Utilisation d'un échantillon dans une mesure de caractéristique électrique selon la revendication 3, dans laquelle les érythrocytes sont fixés de telle sorte qu'une valeur de caractéristique prédéterminée d'une caractéristique électrique d'un mélange des érythrocytes fixés et de composants plasmatiques à une fréquence prédéterminée restera à l'intérieur d'une fourchette de ± 20 % pendant 25 jours après fixation.

5. Utilisation d'un échantillon dans une mesure de caractéristique électrique selon la revendication 4, dans laquelle les érythrocytes sont fixés de telle sorte qu'une différence entre des valeurs maximale et minimale de permittivité d'un mélange des érythrocytes fixés et de composants plasmatiques mesurées à une fréquence de 10 MHz dans le temps restera à l'intérieur d'une fourchette de ± 20 % pendant 25 jours après fixation.

6. Utilisation d'un échantillon dans une mesure de caractéristique électrique selon la revendication 1, dans laquelle le fixateur comprend du glutaraldéhyde.

7. Utilisation d'un échantillon dans une mesure de caractéristique électrique selon la revendication 1, l'échantillon comprenant en outre une solution de MAP.

8. Procédé de mesure de caractéristique électrique comprenant : la mesure d'une caractéristique électrique d'un échantillon à utiliser dans une mesure de caractéristique électrique dans le temps avant la mesure d'une caractéristique électrique d'un échantillon de sang dans le temps, dans lequel
l'échantillon à utiliser dans une mesure de caractéristique électrique comprend des érythrocytes incomplètement fixés avec un fixateur, et les érythrocytes fixés sont tels qu'une caractéristique électrique d'un mélange des érythrocytes fixés et de composants plasmatiques peut varier dans le temps.

9. Dispositif de mesure de caractéristique électrique comprenant : une unité de détermination configurée pour déterminer une précision de mesure sur la base d'une valeur de référence prédéterminée et de valeurs mesurées basées sur une caractéristique électrique mesurée dans le temps au moyen d'un échantillon à utiliser dans une mesure de caractéristique électrique, dans lequel
l'échantillon à utiliser dans une mesure de caractéristique électrique comprend des érythrocytes incomplètement fixés avec un fixateur, et les érythrocytes fixés sont tels qu'une caractéristique électrique d'un mélange des érythrocytes fixés et de composants plasmatiques peut varier dans le temps.
